# EUROPEAN PATENT APPLICATION

(11) **EP 3 437 636 A1**
(43) Date of publication of application: **06.02.2019**
(21) Application number: 17461579.9
(22) Date of filing: 02.08.2017
(51) Int. Cl.: A61K 9/20, A61K 31/00

(54) **PHARMACEUTICAL COMPOSITION COMPRISING EZETIMIBE**

(71) Applicant: Adamed sp. z o.o., 05-152 Czosnów k/Warszawy (PL)
(72) Inventor: WO NIAK, ukasz, 05-152 Czosnów (PL)
(74) Representative: Sulikowski, Daniel

(57) **Abstract**

The invention relates to a pharmaceutical composition comprising 5 to 20 %wt. ezetimibe, 5 to 20 %wt. plastic excipient, 3 to 10 %wt. disintegrant, 1 to 12 %wt. starch, 0.5 to 1.5 %wt. silica, 0.5 to 2.5 %wt. stearic acid, wherein a weight ratio stearic acid to silica is 1:1 to 3: 1, and wherein the pharmaceutical composition of the invention comprises a further diluent and binder, and optionally surfactant. Furthermore, the invention relates to a tablet comprising the composition, and a use of the composition for manufacturing the tablet.

## Description

Ezetimibe belongs to a class of lipid-lowering compounds that selectively inhibits the intestinal absorption of cholesterol and related phytosterols. The chemical name of ezetimibe is 1-(4-fluorophenyl)-3(R)-[3-(4-fluorophenyl)-3(S)-hydroxypropyl]-4(S)-(4-hydroxyphenyl)-2-azetidinone.

Ezetimibe is sold under the brand name Zetia®, which is marketed by Merck/Schering-Plough Pharmaceuticals. Zetia® is available as a tablet for oral administration and is said to contain 10 mg of ezetimibe. The inactive ingredients of Zetia are reported to be croscarmellose sodium, lactose monohydrate, magnesium stearate, microcrystalline cellulose, povidone, and sodium lauryl sulphate. The recommended dose is 10 mg once daily, administered with or without food, according to the Zetia label.

Ezetimibe is reported to be practically insoluble in water. When a solid dosage form comprising ezetimibe is taken orally, the drug must dissolve in aqueous gastrointestinal fluids, e.g., in the patient's stomach, before it can exert a therapeutic effect. A recurring problem with compressed solid oral dosage forms, such as tablets, is that the dissolution rate of the drug limits its biological availability. Since Tₘₐₓ for ezetimibe (in glucuronate form) is relatively short, 1 to 2 hours, it is of utmost importance to assure very fast disintegration of the tablet.

This can be done by the incorporation of what are known as 'water entry promoters' and disintegrants into a tablet. Water entry promoters are substances that promote water entry into a dosage form such as a tablet. This occurs mostly by the transmission of water along the fibres of the promoters. When water reaches the disintegrant, it causes it to swell and the result is the disintegration of the tablet.

One such type of promoters are plastic excipients; characterised by plastic deformation during the compression process. During compression their particles deform plastically to accommodate induced stress. Density of the tablet bed increases with increasing pressure because deforming materials distort to fill void spaces between particles and may also exhibit a tendency to fill the gaps by percolation when the limit of plastic deformation is reached. Plastic excipients have another advantage. They are added to tablets in order to increase their resistance. However, they may worsen tablet blend flowability, and, in consequence, the reproducibility of tableting.

Good flowing properties are essential for achieving reproducible tablet mass. Insufficient blend flow properties can cause high tablet mass variation. The consequence is the variation of the dose available for the patient and subsequently undesired fluctuations of drug blood plasma concentration.

Tablet resistance can be characterised by several parameters such as the absence of tablet capping, tablet lamination or tablet friability. Also robust processes can be achieved by ensuring low tablet ejection force.

Tablet capping and lamination are unacceptable attributes of the formulation, and it is critical to the dose available to the patient (failure to administer a whole dose) and during the processing of the product in a production plant. Therefore, it is necessary to carry out the compression process at lower speeds, which is disadvantageous from an economic point of view. In addition, defective tablets can impair blistering by jamming in the channels, additionally prolonging the manufacturing process. Laminated or capped tablets can be classified as correct by automatic video control of incorrectly filled blisters and then undetected, released and given to the patient. Tablet friability is of similar importance. High tablet friability can generate significant dust during the blistering process, impairing the adhesive properties of the adhesive layer of the cover foil, causing stability issues.

Tablet ejection force is associated with the lubricating properties of some excipients. Additionally, high ejection force can cause sticking to the punches or the undesirable adhesion of the tablet blend to punches, and in consequence, an inappropriate appearance, capping or lamination. In prior art, there are many patents and applications covering ezetimibe in several different pharmaceutical compositions; for example EP 0720599 B, EP 1353696 B, EP 1849459 A, US 2007/0275052 A, EP 2 229 938 B, EP 2 965 752 A, and EP 2 217 214 A2. But none of them address the problem of tablet capping, lamination and friability, as well as increasing tableting yield without an increase in tablet capping, lamination and friability. The problem of dose uniformity is also not addressed.

Therefore, there is a need in the art to provide pharmaceutical composition comprising ezetimibe that enables the obtainment of tablets comprising plastic excipients in relatively small amounts with a high tableting yield and at the same time with very low tablet capping, lamination and friability, as well as very low tablet weight variations.

Thus, the subject of the invention is a pharmaceutical composition comprising:
a) 5 to 20%wt. of ezetimibe,
b) 5 to 20%wt. of a plastic excipient,
c) 3 to 10 %wt. of a disintegrant,
d) 1 to 12%wt. of a starch,
e) 0.5 to 1.5%wt. of a silica, and
f) 0.5 to 2.5%wt. of stearic acid,
wherein a weight ratio stearic acid to a silica is 1:1 to 3:1,
wherein the pharmaceutical composition of the invention comprises a further diluent and binder, and optionally a surfactant. Preferably, the pharmaceutical composition comprises a further diluent, a binder and a surfactant. One skilled in the art would understand that the amount of all additional substances can vary, and the choice of suitable ingredients lies within the skills of one skilled in the art.

The pharmaceutical composition of the invention comprises 5 to 20 %wt. of ezetimibe. The ezetimibe present in the pharmaceutical composition of the invention can have any particle distribution parameter d90. However, since ezetimibe has very low solubility in water, it is preferable in a micronized form to achieve proper bioavailability. Therefore, ezetimibe can have a d90 of less than 25 microns, more preferably less than 20, and even more preferably less than 10 microns. In its most preferable embodiment, ezetimibe has a d90 in a range of 3 to 9 µm to achieve correct bioavailability.

A Malvern Laser Diffraction instrument, equipped with a Hydro 2000S dispersion unit is used to characterise the particle size of ezetimibe. The suspension of the substance is prepared in 1.0% (w/v) Tween 80 water solution by vortex for 10 seconds and by sonication for 45 seconds. The measurement is started after 120 seconds of recirculation after suspension addition into the measurement cell at the speed of 2000 rpm. The other parameters of analysis are as follows: measuring range - 0.02 to 2000 µm, analysis model - general purpose, sensitivity - normal and particle shape - irregular.

The pharmaceutical composition of the invention comprises 5 to 20% wt. of a plastic excipient. The 'plastic excipient' is characterised by plastic deformation during the compression process. During compression particles deform plastically to accommodate induced stress. Density of the tablet bed will increase with increasing pressure because deforming materials will distort to fill void spaces between particles and may also exhibit a tendency to fill the gaps by percolation when the limit of plastic deformation is reached. As a preferred embodiment, the plastic excipient is selected from microcrystalline cellulose, powdered cellulose, cyclodextrins, preferably beta and gamma cyclodextrins, low-substituted hydroxypropyl cellulose, and maltodextrin. In its most preferable embodiment, the plastic excipient is selected from microcrystalline cellulose and low-substituted hydroxypropyl cellulose.

The pharmaceutical composition of the invention also comprises 3 to 10 %wt. of disintegrant. The disintegrant is selected from carboxymethylcellulose calcium, carboxymethylcellulose sodium, croscarmellose sodium, crospovidone, magnesium aluminium silicate, polacrillin potassium, cross-linked polyvinylpyrrolidone, sodium alginate and sodium starch glycolate. Preferably, the disintegrant is croscarmellose sodium or cross-linked polyvinylpyrrolidone.

The pharmaceutical composition of the invention comprises 1 to 12%wt. of starch. Preferably, the starch contained in the pharmaceutical composition is a pregelatinised starch. The pregelatinised starch can be for example pregelatinised maize starch or pregelatinised potato starch.

The pharmaceutical composition of the invention comprises 0.5 to 1.5%wt. of a silica. Preferably, the silica is a colloidal silica. In a preferred embodiment, the colloidal silica has a specific surface area in the range of 200 to 300 m2/g. The use of a colloidal silica with a specific surface area below 200 m2/g leads to the deterioration of the properties of the final tablets.

The pharmaceutical composition of the invention can comprise 20 to 80 %wt. of a further diluent. The diluent is selected from calcium carbonate, calcium sulphate, sugar, dextrates, dextrin, dextrose, dibasic calcium phosphate dihydrate, tribasic calcium phosphate, lactose monohydrate, magnesium carbonate, magnesium oxide, maltodextrin, mannitol, lactose anhydrous, sorbitol and talc. Preferably, the further diluent is lactose or mannitol.

The pharmaceutical composition of the invention can comprise 1 to 10 %wt. of binder. The binder is selected from acacia, alginic acid, carbomer (e.g. carbopol), carboxymethylcellulose sodium, copovidone (Plasdone® S-630), dextrin, ethyl cellulose, gelatine, guar gum, hydroxyethyl cellulose, hydroxypropyl cellulose, hydroxypropyl methyl cellulose, liquid glucose, maltodextrin, methylcellulose, polymethacrylates, polyvinylpyrrolidone (PVP, e.g., povidone), pregelatinised starch, sodium alginate and starch. Preferably, the binder is polyvinylpyrrolidone and hydroxypropylcellulose.

The pharmaceutical composition of the invention can comprise 0-5 %wt., of surfactant. The surfactant selected is sodium lauryl sulphate (SLS).

Another subject of the invention is a tablet comprising the pharmaceutical composition of the invention.

The most commonly used method for preparing a solid dosage form such as a tablet is a wet granulation method. Techniques used in the method are very well known in the art.

In wet granulation, some or all of the active ingredients and excipients in powder form are blended and then further mixed in the presence of a liquid, typically water or an aqueous binder solution, which causes the powders to clump up into granules. The granulate is screened and/or milled, dried and then screened and/or milled to the desired particle size. The granulate can then be tableted or other excipients can be added prior to tableting, such as a glidant and/or a lubricant. Another wet granulation technique involves the production of granules by spraying water or an aqueous binder solution onto the fluidising powder bed in an atmosphere of dry air at an elevated temperature. The resulting granules can be screened and/or milled and blended with other excipients and tableted.

Wet granulation is often preferred over direct compression because it is more likely that wet granulation overcomes the problems associated with the physical properties of the individual components in the formulation, such as a low solubility, poor flowing or sticking to the punches. Wet granulation provides a material that has the required flow and cohesive properties necessary to obtain an acceptable solid dosage form, i.e. tablet. Tablet uniformity is generally improved with wet granulation because all of the granules usually contain the same amount of drug. It also avoids separation of the drug from the excipients.

In a preferred embodiment, the tablet is obtained using a wet granulation method. It was shown that the use of a wet granulation method in connection with the pharmaceutical composition of the invention results in tablets with not only very good resistance to capping and lamination, but it can also ensure good tablet friability and high reproducibility. Applied formulation gives the product uniformity, by reducing mass variation between tablets without having to compromise it with the reduction of tableting process efficacy.
Still another aspect of the invention relates to the use of the pharmaceutical composition for the manufacture of tablets comprising ezetimibe. Preferably, the tablet is manufactured using a wet-granulation method.

### EXAMPLES

Parameters used in the context of the characterisation of a tablet obtained from the pharmaceutical composition of the inventions can be measures as follows:

### a) Tablet friability.

Test conducted on tablet friability apparatus, in accordance with Ph. Eur. 2.9.7 'Friability of uncoated tablets'.

### b) Tablet hardness.

Test conducted on apparatus consisting 2 jaws facing each other. Tablets placed between jaws are crushed and the force used is measured. Test in accordance with Ph. Eur. 2.9.8 'Resistance to crushing of tablets'.

### c) Appearance test

After the friability test, all tablets are inspected. Tablets which exhibit unfavourable splitting (lamination and capping) from the applied force during compression are counted and calculated as x tablets per 1000 tablets.

### d) Similarity factor.

Similarity factor calculated according to EMA guideline: Appendix I to the 'GUIDELINE ON THE INVESTIGATION OF BIOEQUIVALENCE'.

### Example 1. Comparison of formulation of the invention with reference formulation

In the developmental study aimed at developing a stable formulation for ezetimibe, it was surprisingly found that tablets containing less excipients with plastic deformation characteristics have a beneficial effect on the rheological properties of the tablet blend. This translates into a significant reduction in tablet weight variability, which provides the opportunity to work at higher efficiencies, over 130,000 tablets per hour, and provides patients with a high quality product.

### Tablet preparation:

Ezetimibe was added to a high shear granulator together with excipients and half of the disintegrant. The plastic excipient, i.e. microcrystalline cellulose was added in one portion (Formulation Nos. 2, 5, or 6) or was divided between intragranular and extragranular fractions (Formulation Nos. 1 or 4). After preblending, the blend was granulated using a water solution of a binder (e.g., Povidone). The resulting granulate was screened, and then the remaining components (from the extragranular phase) were added. Separated blending was used before the lubricant was added, and after adding it. Formulation 3 was manufactured by the granulation of ezetimibe with lactose; 2/3 part of the maize starch and half of the disintegrant, using a binder solution and subsequently other excipients were admixtured before tableting.

Tableting was performed using a rotary tablet press and tablets were formed as capsule- shaped tablets with dimensions of 8x5 mm.

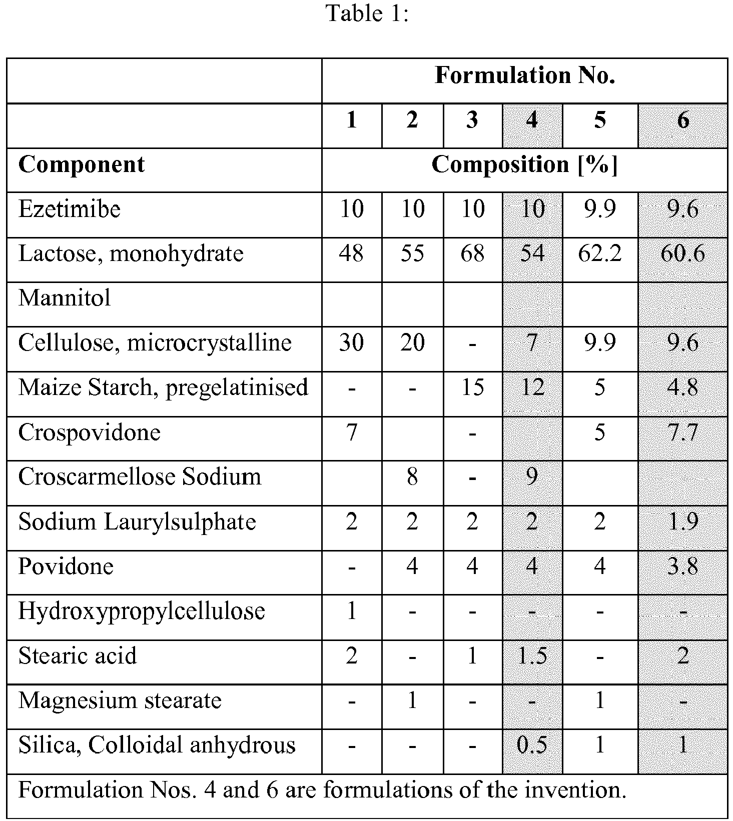

The results obtained for the above formulations are presented in Table 2.

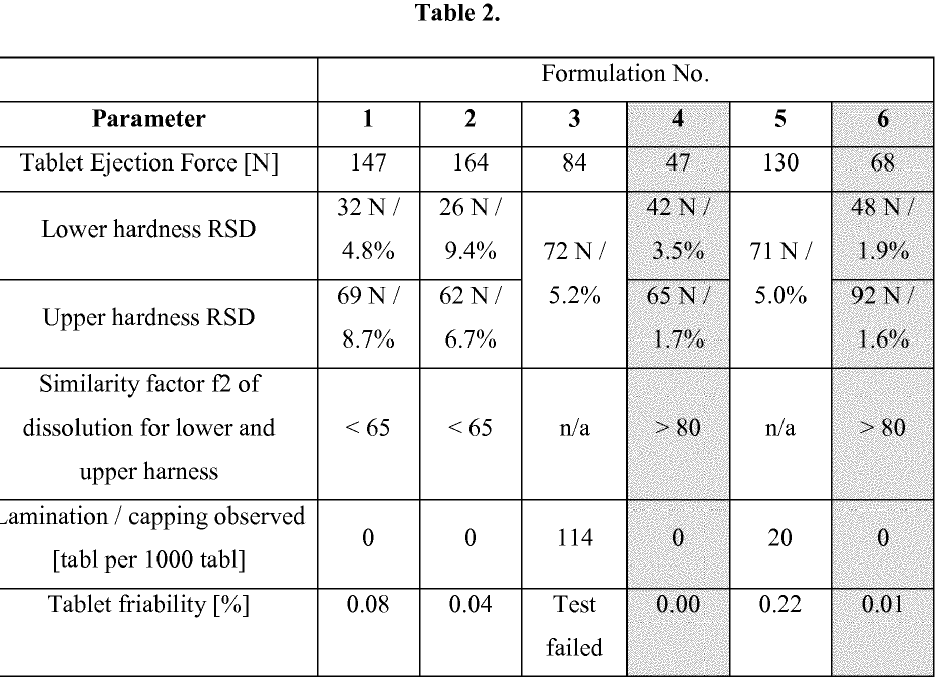

Formulations with a significant amount of excipients with plastic characteristics due to their composition have very high mechanical resistance, which is a direct result of the properties of the raw materials used. Reference Formulations 1 and 2, which contain at least 20% of plastic excipients have relatively high ejection forces which may adversely affect the tableting process during the production of a larger volume with, for example, the undesirable adhesion of the tablet blend to tablet punches and dies and faster tool wear. Furthermore, the tablet blends prepared according to the aforementioned compositions, have worse flow characteristics, which results in a greater tablet mass RSD. This translates directly into a greater fluctuation in the amount of active substance in individual doses. This effect increases with higher tableting performance. Lower machine speeds required due to an absence of tablet weight uniformity will result in the prolongation of the manufacturing process and thus increase manufacturing costs. Reference Formulation 3, in which stearic acid, maize starch and colloidal silica are not present at the same time, results in problems with the mechanical resistance of the tablets in the friability test. A large number of unacceptable tablets were also observed (capping or lamination). Formulation 4 of the invention is characterised by high resistance to damage and low ejection forces. Good flow properties of the tableting blend result in a much more homogeneous product. It is thus possible to conduct the tableting process at the higher operating ranges, reducing time and costs.

Reference Formulation 5, and Formulation 6 of the invention illustrate the effect of the lubricant used (magnesium stearate vs. stearic acid). Unexpectedly, the result was that the replacement of magnesium stearate with stearic acid significantly improves tablet resistance to capping/lamination. This effect reduces the ejection force and thus friction of the compressed tablet against the matrix walls.

Furthermore, it was surprisingly noticed, that formulations of the invention compressed with lower or higher compression forces result in a robust product in terms of the reproducibility of dissolution profiles. Formulations with higher level of plastically deforming excipients are compression dependent (Formulation 1 and 2), and their dissolution profiles vary in accordance to the pressure applied during compression. Formulations of the invention (Formulation 4 and 6) represent very high similarity factor, which guarantees the same release of the API in the human body.

### Example 2. Composition containing more colloidal silica than stearic acid

Reference Formulation 7 was prepared in order to investigate the effect of a quantitative excess of colloidal silica over stearic acid. The example has a stearic acid to silica ratio of 0.6:1.

### Tablet preparation:

Ezetimibe was added to a high shear granulator together with SLS, a half of the croscarmellose and a half of the mannitol and microcrystalline cellulose. After preblending, the mixture was granulated using an aqueous solution of Povidone. The resulting granulate was dried and homogenised, and then mixed with the rest of the components, i.e. croscarmellose, mannitol, microcrystalline cellulose, starch, colloidal silica and stearic acid. Separated blending was used before the lubricant was added, and after adding it.

Tableting was done using a rotary tablet press and tablets were formed as capsule shaped tablets with dimensions of 8x5 mm.

| | **Formulation No. 7** |
|---|---|
| **Component** | **Composition [%]** |
| Ezetimibe | 10 |
| Mannitol | 51 |
| Cellulose microcrystalline | 15 |
| Maize Starch, Pregelatinised | 6 |
| Croscarmellose Sodium | 8 |
| Sodium lauryl sulphate | 2 |
| Povidone | 4 |
| Stearic acid | 1.5 |
| Silica, Colloidal anhydrous | 2.5 |

The results obtained for the above formulations are presented in the following table.

| **Parameter** | **Formulation 7** |
|---|---|
| Mean tablet hardness [N] | 62 |
| Hardness relative standard deviation [%] | 8.5 |
| Tablet Ejection Force [N] | 276 |
| Tablet friability [%] | 0.17 |
| Lamination/ capping observed [tabl per 1000 tabl] | 20 |

The result was that while the quantity of starch does not affect the characteristics of the tablet prepared according to the invention, the ratio of stearic acid to colloidal silica is important. It was shown that the ratio of stearic acid to colloidal silica should be in a range of 1:1 to 3:1 in favour of stearic acid. Reference Formulation 7 shows an example where the above ratio is outside of that range. In the aforementioned example, the ejection forces increase dramatically, making it impossible to conduct the process. Formulations with a higher excess of stearic acid are undesirable.

### Example 3. Exemplary formulations of the invention

### Tablet preparation:

### Preparation of tablet bled for Formulation Nos. 8, 10, and 11.

Ezetimibe was added to a high shear granulator, together with SLS, and a half of the disintegrant and diluents. In Formulation 8, the plastic excipient is in the extragranular fraction, while in both Formulation 10 and 11, a half of the plastic excipient is in the extragranular fraction, and the other half is in the intragranular fraction. After preblending, the blend was granulated using a water solution of a binder (e.g., Povidone or Hydroxypropylcellulose). The resulting granulate was homogenised, and then the remaining components (of the extragranular fraction) were added. Separated blending was used before the lubricant was added, and after adding it.

Formulation 9 was prepared using fluid bed granulation. All granulate ingredients were introduced into the chamber of the device and granulated using a binder solution during fluidisation. SLS, half of the lactose, colloidal silica and starch were added to the resulting granulate, and after blending, stearic acid.

Tableting was done using a rotary tablet press and tablets were formed as capsule shaped tablets with dimensions of 8x5 mm.

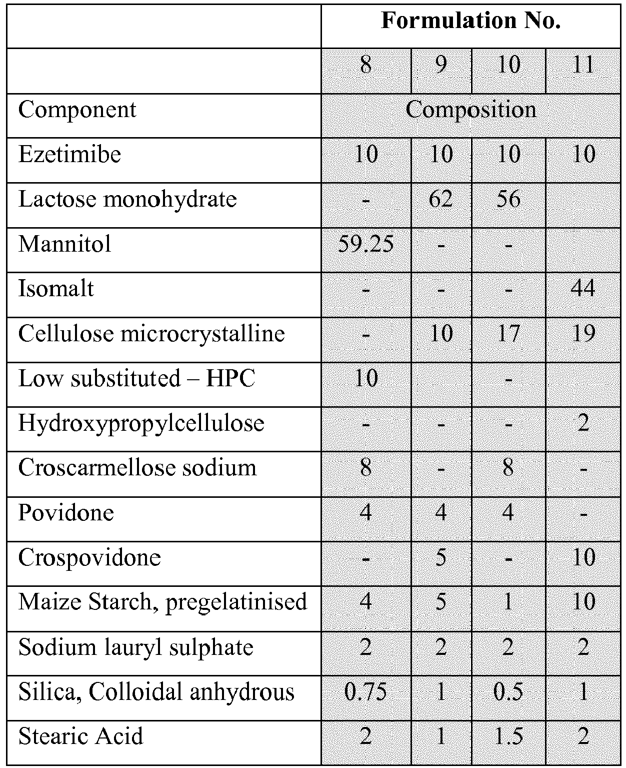

The results obtained for the above formulations are presented in the table below.

| | **Formulation No.** | | | |
|---|---|---|---|---|
| **Parameter** | **Formulation 8** | **Formulation 9** | **Formulation 10** | **Formulation 11** |
| Lower hardness RSD | 31 N / 2.7% | - | 65 N / 1.3% | 40 N / 3.7% |
| Upper hardness RSD | 81 N / 2.5% | 91 N / 2.8% | 72 N / 3.9% | 80 N / 2.9% |
| Tablet Ejection Force [N] | 45 | 64 | 75 | 32 |
| Lamination/ capping observed [tabl per 1000 tabl] | 0 | 0 | 0 | 0 |
| Tablet friability [%] | 0.03 | 0.05 | 0.07 | 0.05 |

The examples above show four formulations of the invention, where different manufacturing process techniques were applied; namely high shear and fluid bed granulation. Product parameters like hardness RSD (related to tablet mass RSD), ejection force, and friability are very low and no lamination/capping occurs.

## Claims

1. A pharmaceutical composition comprising:
a) 5 to 20 %wt. ezetimibe,
b) 5 to 20 %wt. plastic excipient,
c) 3 to 10 %wt. disintegrant,
d) 1 to 12 %wt. starch,
e) 0.5 to 1.5 %wt. silica,
f) 0.5 to 2.5 %wt. stearic acid,
wherein a weight ratio stearic acid to silica is 1:1 to 3:1,
wherein the pharmaceutical composition of the invention comprises a further diluent and binder, and optionally surfactant.

2. Pharmaceutical composition according to claim 1, wherein the particle size distribution for ezetimibe d90 is in a range of 3 to 9 µm.

3. Pharmaceutical composition according to any of claims 1 to 2, wherein the plastic excipient is selected from microcrystalline cellulose, powdered cellulose, cyclodextrins, low-substituted hydroxypropyl cellulose, and maltodextrin.

4. Pharmaceutical composition according to claim 3, wherein the plastic excipient is selected from microcrystalline cellulose, and low-substituted hydroxypropyl cellulose.

5. Pharmaceutical composition according to any of claims 1 to 4, wherein the disintegrant is selected from carboxymethylcellulose calcium, carboxymethylcellulose sodium, croscarmellose sodium, crospovidone, magnesium aluminium silicate, polacrillin potassium, cross-linked polyvinylpyrrolidone, sodium alginate and sodium starch glycolate

6. Pharmaceutical composition according to any of claims 1 to 5, wherein the starch is a pregelatinised starch.

7. Pharmaceutical composition according to any of claims 1 to 6, wherein the silica is a colloidal silica.

8. Pharmaceutical composition according to claim 7, wherein the colloidal silica has a specific surface area in the range of 200 to 300 m²/g.

9. Tablet comprising a pharmaceutical composition as defined in any of claims 1 to 8.

10. Tablet according to claim 9, wherein the tablet is manufactured using a wet-granulation method.

11. Use of pharmaceutical composition as defined in any of claims 1 to 8 for the manufacture of tablet comprising ezetimibe.

12. Use according to claim 11, wherein the tablet is manufactured using a wet-granulation method.
